(19) Europäisches Patentamt
European Patent Office
Office européen des brevets

(11) Publication number: 0 232 423 A1

(12) # EUROPEAN PATENT APPLICATION

published in accordance with Art. 158(3) EPC

(21) Application number: 86904371.1

(22) Date of filing: 09.07.86

Data of the international application taken as a basis:

(86) International application number: PCT/JP 86/00350

(87) International publication number: WO 87/00433 (29.01.87 87/3)

(51) Int. Cl.⁴: A 61 K 31/445, C 07 D 211/14

(30) Priority: 24.07.85 JP 162128/85
31.07.85 JP 167727/85
31.07.85 JP 167728/85
28.08.85 JP 187482/85
12.11.85 JP 252069/85

(43) Date of publication of application: 19.08.87 Bulletin 87/34

(84) Designated Contracting States: CH DE FR GB LI

(71) Applicant: GRELAN PHARMACEUTICAL CO., LTD., 3-9 Nozawa 3-chome Setagaya-ku, Tokyo 154 (JP)

(72) Inventor: KIGASAWA, Kazuo, 15-7-701, Nozawa 4-chome, Setagaya-ku Tokyo 154 (JP)
Inventor: IRINO, Osamu, 9-1, Horinouchi 1-chome, Suginami-ku Tokyo 166 (JP)
Inventor: HONDA, Hideo, 13-5-704, Takaban 3-chome, Meguro-ku Tokyo 152 (JP)
Inventor: OOBA, Seiichi, 9-13, Umejima 3-chome, Adachi-ku Tokyo 121 (JP)
Inventor: SAITO, Kiyoshi, 32-18, Sangenjaya 1-chome, Setagaya-ku Tokyo 154 (JP)
Inventor: UCHIDA, Katsuyuki, 2418-12, Samukawamachi Ichinomiya, Kouza-gun Kanagawa 253-01 (JP)

(74) Representative: Bourgognon, Jean-Marie et al, Cabinet Flechner 22, Avenue de Friedland, F-75008 Paris (FR)

(54) **CIRCULATION IMPROVING AGENT.**

(57) A circulation improving agent containing 2-(4-benzylpiperidino)-1-(4-benzyloxyphenyl)propan-1-ol or a salt thereof as an effective ingredient can be used as a brain circulation improving agent, vasodilator, coronary vasodilator, calcium antagonist, antiplatelet agent, or $\alpha_1$-blocker for prophylaxis, treatment, and improvement of circulatory troubles or diseases.

EP 0 232 423 A1

SPECIFICATION

# CARDIOVASCULAR DRUG

## Technical Field

The present invention relates to a cardiovascular drug comprising 2-(4-benzylpiperidino)-1-(4-benzyloxyphenyl)propan-1-ol or its salt as the active ingredient.

## Background Art

2-(4-benzylpiperidino)-1-(4-benzyloxyphenyl) propan-1-ol, which is the active ingredient of the drug of the present invention is the compound disclosed in Japanese Patent Unexamined Publication Nos. 55-28903, 56-79666 and 56-79667, but nothing was known about its pharmaceutical effects. Furthermore, its related compounds are disclosed, for example, in Japanese Patent Examined Publication Nos. 47-15348 and 58-24434.

In recent years, especially with the advance in the ages of people, changes in eating habits, etc., there has been a trend toward an increase in cardiovascular diseases, and their treatment and prophylaxis have become an important problem in medical care. Hitherto, when abnormal symptoms occur, for example, (a) when obstruction of blood circulation occurs due to the stricture or occlusion of blood vessels, a vasodilator has been used; (b) when the supply of blood to the myocardium is blocked due to stricture or occlusion of the coronary artery, the resultant ischemic state gives rise to coronary insufficiency, and in such cases, vasodilation of the coronary artery and increase of coronary blood flow volume by the administration of a coronary vasodilator (which has the

essential effect of dilating the smooth muscle of the coronary artery) has been prescribed; (c) a calcium (Ca) antagonist has been used for the treatment of angina pectoris (electrical excitement of the myocardium and smooth muscles is induced by extra-cellular calcium ions increasing the intra-cellular calcium level by passing through the slow-channel in cell membranes and the Ca antagonist interferes with this slow-channel, resulting in the inhibition of myocardia contraction and relaxation of the smooth muscles); (d) it has been said that lesions in the wall of blood vessels cause platelets to become adhesive and to aggregate, resulting in the formation of thrombi, and in such cases, an anti-thrombotic drug has been used; (e) for the treatment of hypertension, diuretic anti-hypertensive drugs for decreasing blood pressure, α- or β-adrenoceptor blockers, vasodilators, calcium antagonists, etc. has been used.

As examples of the above drugs, nitroglycerine, isosorbide dinitrate, verapamil, nifedipine, nicardipine, diltiazem, propranolol, ifenprodil, prazosin, ticlopidine, calcium hopantenate may be cited. However, these drugs have problems such as undesirable side effects, limited scope of clinical effect, slowness in achieving clinical effect, inadequate duration of clinical effect, difficulty in controlling the effect due to excessive potency, etc.

Recently, the development of new α-adrenoceptor blockers is attracting special interest. As α-adrenoceptor, the existence of $\alpha_1$- and $\alpha_2$-adrenoceptors has been confirmed and their roles are now being clarified. That is, when the $\alpha_1$-adrenoceptor is stimulated, peripheral resistance increases to induce increases in blood pressure; while when $\alpha_2$-adrenoceptor is stimulated, the release of norepinephrine, which

acts hypertensively, is inhibited. Thus, a substance can be envisaged which selectively inhibits the $\alpha_1$-adrenoceptors as against the $\alpha_2$-adrenoceptors for use as an anti-hypertensive drug. Most of known α-adrenoceptor blockers have no such specificity and act on both the $\alpha_1$- and $\alpha_2$-adrenoceptors and are therefore difficult to use as an anti-hypertensive drug.

At the same time, there is also a demand for the development of new drugs which can improve cerebral circulation or cerebral metabolism by acting more selectively on the brain than on the peripheral organs.

Disclosure of Invention

The object of the present invention is to provide a drug needed as a cardiovascular drug which is free from the above problems seen in conventional drugs.

During the development and research for new circulatory drugs under the above-mentioned technical circumstances, the inventors discovered that 2-(4-benzylpiperidino)-1-(4-benzyloxyphenyl)propan-1-ol [sometimes called "compound (I)" hereinafter] acted effectively on the circulatory system and accomplished the present invention after further research.

That is, the present invention relates to a cardiovascular drug containing compound (I) or its salt as the effective ingredient.

Compound (I) is a compound having the following structure.

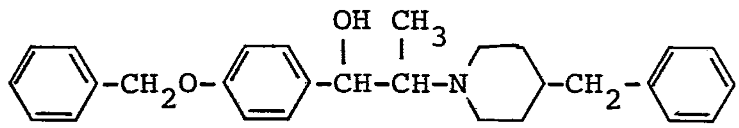

(I)

Compound (I) includes both isomers of erythro form and threo form. In the present invention, both of them can be used, but normally the erythro form is more preferably used.

Salts of compound (I) include pharmaceutically acceptable salts of organic acids and inorganic acids. As such salts of organic acids, mention may be made of, for example, the salts of aliphatic carboxylic acids having 2-20 carbon atoms such as tartaric acid, lactic acid, acetic acid, malic acid, citric acid, fumaric acid, maleic acid, succinic acid, palmitic acid, stearic acid, oleic acid and linoleic acid; and the salts of aliphatic or aromatic sulfonic acids having 1-10 carbon atoms such as p-toluenesulfonic acid, methanesulfonic acid and naphthalensulfonic acid. Typical examples of said salts of inorganic acids are salts of inorganic mineral acids such as hydrochloric acid, hydrobromic acid, phosphoric acid and sulfuric acid.

Compound (I) can be manufactured, for example, by the process disclosed in Japanese Patent Unexamined Publication No. 55-28903. Salts of compound (I) can be prepared in accordance with the conventional process by reacting compound (I) with the above-mentioned acids in a solvent (e.g., ethanol, methanol, benzene, acetone, chloroform, water).

As the drug of the present invention, compound

(I) or its salt may be used as it is, but usually it is preferred to use it with the addition of pharmacologically or pharmaceutically acceptable excipients. When excipients are used, the combination ratio of compound (I) in the drug of the present invention is normally 0.1-10% by weight and preferably 0.2-5% by weight.

As said excipients, such known pharmaceutical substances as, for example, those disclosed in A.O. Chairman et al's "Remington's Pharmaceutical Science" (published by Mack Publishing Co. USA, in 1980) may be optionally used. Specifically, excipients for pharmaceutical compositions are chosen which are suitable for the intended routes of administration such as oral dosage form, injections, mucosal dosage form (buccal, troche, suppository, etc.) and transdermic dosage form (ointment, patch, etc.). These pharmaceutical excipients are used as, for example, fillers (e.g., starch, lactose), disintegrators (e.g., carboxymethyl cellulose), lubricants (e.g., magnesium stearate), coating agents (e.g., hydroxyethyl cellulose) and corrigents for oral dosage form and mucosal dosage form; solvents or solubilizing agents capable of forming aqueous injections (e.g., distilled water for injection, normal saline solution, propylene glycol), suspending agents (e.g., surfactants such as polysorbate 80), pH adjustment agents (e.g., organic acids or metallic salts thereof) and stabilizers for injections; aqueous or oily solvents or solubilizing agents (e.g., alcohols, fatty acid esters), stickers (e.g., carboxyvinyl polymers, polysaccharides) and emulsifying agents (e.g., surfactants) for transdermic dosage form.

Various processes may be employed for the preparation of the drug of the present invention so long

as the object of the present invention is not obstructed. In general, they can be prepared by processes known per se [e.g., the process described in the general rules for preparation of The Pharmacopoea of Japan 10th edition (JPX) or by means utilized for production of foods and candies] or processes with adequate modifications given to these known processes or means.

The improvement of the circulatory system according to the present invention means improvement of the state of diseases in the organs and tissues which are involved in the circulation of blood and more specifically, includes improvement, prophylaxis and treatment of lesions or diseases in organs and tissues involved in circulation such as the brain, heart, liver, kidney, central or peripheral blood vessels, etc. by acting on the structure or function of these organs and tissues. In some cases, especially in the case of cerebral disorders, improvement of circulation may bring about secondary or supplementary improvements in the activation of the metabolic system.

The drug of the present invention has the activity of improving circulation as mentioned above in mammals such as human, monkey, dog, cat, rabbit, rat, mouse, etc. and so can be effectively used as cardiovascular drugs. That is, the drug of the present invention has various activities such as improvement of cerebral circulation, vasodilative action, coronary vasodilative action, calcium antagonistic action, anti-aggregative action on platelets and $\alpha_1$-adrenoceptor blocking action ($\alpha_1$-blocking action) and thus can be used as a cardiovascular drug. Therefore, a more specific representation of the present invention as a cardiovascular drug may be as, for example, a cerebral circulation improving drug (cerebrovascular drug),

vasodilator, coronary vasodilator, calcium antagonist, anti-platelet drug (anti-platelet aggregation drug), $\alpha_1$-blocker, anti-thrombotic drug, anti-hypertensive drug and cerebral metabolism improving or activating agent for humans.

As to diseases for which the drug of the present invention is used, mention may be made of angina pectoris, effort angina, arrhythmia, hypertension, spontaneous hypertension, coronary atherosclerosis, atherosis, acute or chronic myocardial infarction, cerebral infarction, cerebral hemorrhage, (transient) cerebral ischemic attacks, endotoxin shock, coronary occlusion, peripheral artery occlusion, pulmonary occlusion, Kawasaki disease, cerebrovascular diseases, cerebral metabolic disease, thromboembolism in humans, etc. The drug of the present invention may be used for prophylaxis, treatment and improvement of these diseases, disorders and lesions or treatment and improvement of their sequelae.

The drug of the present invention, namely, compound (I) or its salt or a preparation with the addition of excipient mentioned above may be administered orally or parenterally (injection, mucosal dosage form or transdermic dosage form).

The dose of the drug of the present invention, for example, for human adult, is 10-300 mg/day and preferably 20-200 mg/day once a day or in divided doses 2-4 times a day in terms of the amount of compound (I) or its salt in case of the oral dosage form and transdermic dosage form. 1/2-1/20 of the above dose is sufficient in case of the injection and mucosal dosage forms. However, it is desirable to administer the drug in optionally increased or decreased doses depending

on the kind and stage of the disease and age and sex of patients.

Next, Examples of pharmacological tests on the drug of the present invention are shown.

Test Example 1

Acute toxicity of compound (I); $LD_{50}$ (mg/kg) > 1200 (mice, oral); > 70 (mice, venous injection).

Compound (I) is a compound of very low toxicity.

Test Example 2

(Experimental method)

Mongrel dogs of either sex (body weight 10-17 kg) were killed by bleeding under anesthetization with pentobarbital sodium (tradename: Nembutal ®, Abbott Co., USA). Thereafter, the basilar and femoral arteries were extracted and attached blood and connective tissues were carefully removed in a dish filled with Krebs bicarbonate solution (composition: NaCl 117.7 mM, KCl 4.7 mM, $CaCl_2$ 2.5 mM, $MgSO_4$ 1.2 mM, glucose 10 mM, $NaHCO_3$ 24.4 mM). Thereafter, ring preparations were made therefrom. This preparation was suspended in a bathing tube of 10 ml filled with Krebs bicarbonate solution aerated with 95 % $O_2$ and 5 % $CO_2$.

Then, samples of basilar and femoral arteries were contracted by application of 50 mM KCl. After confirming that the contraction was sustained, (a) compound (I) (erythro form) lactate, (b) compound (I) (threo form) lactate and (c) ifenprodil; [2-(4-benzyl-piperidino-1-(4-hydroxyphenyl)propane-1-ol] tartrate were, respectively, administered as test compounds and the dose-relaxation response was examined.

A strain gauge transducer (model SB-1T manufactured by Nihon Koden Co.) was employed for recording of change of tension and isometric contraction was recorded on a recorder (model QPD 53 made by Hitachi Ltd.).

(Results and discussion)

The results are shown in Tables 1 and 2.

Table 1 Relaxation of isolated basilar artery (mean value %, n = 4 - 5)

| Dose / Compound | $10^{-8}$ M | $10^{-7}$ M | $10^{-6}$M | $10^{-5}$ M | 5 x $10^{-5}$ M | $10^{-4}$ M | $ED_{50}$ |
|---|---|---|---|---|---|---|---|
| (a) Compound (I) (erythro form) lactate | 7.0 | 39.2 | 100 | - | - | - | 1.6 x $10^{-7}$ M |
| (b) Compound (I) (threo form) lactate | 10.8 | 72.1 | 91.7 | 100 | - | - | 8.5 x $10^{-8}$ M |
| (c) Ifenprodil tartrate | | 0.9 | 5.1 | 44.1 | 96.6 | 100 | 1.1 x $10^{-5}$ M |

Table 2 Relaxation of isolated femoral artery (mean value %, n = 4 - 5)

| Compound \ Dose | $10^{-8}$ M | $10^{-7}$ M | $10^{-6}$ M | $10^{-5}$ M | $5 \times 10^{-5}$ M | $10^{-4}$ M | $ED_{50}$ |
|---|---|---|---|---|---|---|---|
| (a) Compound (I) (erythro form) lactate | 0.4 | 1.2 | 34.2 | 59.9 | - | 85.8 | $7.2 \times 10^{-6}$ M |
| (b) Compound (I) (threo form) lactate | - | 0 | 6.8 | 22.5 | - | 62.3 | $5.1 \times 10^{-5}$ M |
| (c) Ifenprodil tartrate | - | 0 | 0 | 2.8 | 49.8 | 75.9 | $5.2 \times 10^{-5}$ M |

The above results show that compound (I) has the activity of relaxing blood vessels in the state of sustained contraction and hence the drug of the present invention can be effectively used as a vasodilator. It has the a potent action on the cerebral artery and peripheral vessels, especially on the cerebral artery, and hence can be used as a cerebrovascular drug.

Test Example 3

(Experimental Method)

Experiments were conducted in accordance with the method of Test Example 2 with use of the coronary artery in place of the basilar and femoral arteries.

(Results and discussion)

The results are shown in Table 3. The degree of relaxation is expressed by the relative value when relaxation response upon application of paraverine ($10^{-4}$ M) is assumed to be 100 %.

Table 3 Relaxation of isolated coronary artery (mean value %, n = 8)

| Dose / Compound | $10^{-6}$ M | $10^{-5}$ M | 5 x $10^{-5}$ M | $10^{-4}$ M | 5 x $10^{-4}$ M | $ED_{50}$ |
|---|---|---|---|---|---|---|
| (a) Compound (I) lactate | 40.6 | 92.1 | - | 100 | - | 2.2 x $10^{-6}$ M |
| (b) Ifenprodil tartrate * 1 | - | 0.1 | 59.6 | 87.6 | 91.7 | 2.6 x $10^{-5}$ M |

* 1 : n = 4

The above results show that compound (I) has the activity of relaxing vessels in the state of sustained contraction and hence the drug of the present invention can be effectively used as a coronary vasodilator.

Test Example 4

(Experimental Method)

The same measurements as in Test Example 2 were carried out using the isolated basilar artery and isolated femoral artery treated in accordance with the method of Test Example 2.

For examination of the Ca antagonistic action, the depolarization of both isolated arteries was induced with 50 mM KCl in a bathing solution from which Ca had been removed and then $CaCl_2$ was cumulatively applied thereto. As test compounds, (a) compound (I) (erythro form) lactate and (b) ifenprodil tartrate were applied at 15 minutes before addition of $CaCl_2$.

(Results and discussion)

The results are shown in Table 4.

Table 4 $pA_2$ values on Ca-induced contraction in depolarized muscle by K. Note 1)

| | Compound (I) lactate Note 2) | Ifenprodil tartrate Note 3) |
|---|---|---|
| Isolated basilar artery | 7.73 | 5.27 |
| Isolated femoral artery | 5.81*** | 4.53** |

Note 1) $pA_2$ value: Iyakuhin Kaihatsu Kiso Koza V "Assessment of Drug (1)", the second volume, page 763, 1971 (published by Chijin Shokan Co.)

Note 2) n = 3 - 4

Note 3) n = 6 *** : P < 0.001 ** : P < 0.01

The above results show that compound (I) exhibits a stronger Ca antagonistic action than ifenprodil. Furthermore, both compounds exhibit competitive antagonism on Ca-induced contraction in depolarized basilar and femoral arteries by K, and the degree of the antagonism is greater on the basilar artery than on the femoral arteries.

The above results also show that the drug of the present invention can be effectively used for above mentioned diseases as a calcium antagonist having an effective action on the cerebral artery and peripheral blood vessel, especially on the cerebral artery, and so can be used as a cerebrovascular drug.

<u>Test Example 5</u>

(i) Activity against thromboembolic respiratory depression induced by ADP.

(Introduction)

Adenosine di-phosphate (ADP) is known to possess a platelet aggregating action and is considered to have an important role in the formation of platelet thrombi ["Thrombo-embolism Kiso to Rinsho" supervised by Mototaka Murakami, pages 29-31, 1977 (published by Japan Medical Center)]. The degree of formation of platelet thrombi in the pulmonary vessels of mice induced

by intravenous administration of ADP can be measured based on the decrease in the respiratory rate. The anti-platelet thrombotic action of test drugs can be evaluated by using this decrease in the respiratory rate as a standard.

(Experimental Method)

Suspensions of the following drugs in 1 % gum arabic (10 ml/kg) were orally administered to ddY male mice (body weight 27 - 30 g).

| | | |
|---|---|---|
| (a) | Compound (I) (erythro form) | 30 mg/kg |
| (b) | Ifenprodil tartrate | 30 mg/kg |
| (c) | Ticlopidine-HCl | 30 mg/kg |
| (d) | Diltiazem-HCl | 10 mg/kg |

As the control drug, 10 ml/kg of 1 % gum arabic was administered in the same manner. After 1/3, 1, 2, 3 and 5 hours from administration, a solution of ADP 40 mg/kg in saline was administered from the caudal vein over a period of 3 - 4 seconds and after lapse of 10 seconds the respiratory rate was measured. Relative respiratory rate (%) was calculated according to the following formula.

$$\text{Relative respiratory rate} = \frac{\text{Respiratory rate 10 seconds after administration of ADP}}{\text{Respiratory rate before administration of ADP}} \times 100\ (\%)$$

(cf. "Thrombosis Research", Vol. 35, Page 311, 1984)

(Results and discussion)

The experimental results are shown in Table 5-1.

Table 5-1 note 1)

| Test compound | Relative respiratory rate (%) Time after administration (hr) 1/3 | 1 | 2 | 3 | 5 |
|---|---|---|---|---|---|
| (a) Compound (I) | 53.1 ±11.7* (n=7) | 51.2 ±5.6* (n=10) | 52.8 ±7.4* (n=10) | 49.0 ±8.4(*) (n=9) | 41.9 ±11.5 (n=8) |
| (b) Ifenprodil tartrate | 34.6 ±10.2 (n=8) | 61.3 ±9.3** (n=6) | 52.7 ±6.1* (n=10) | 41.0 ±4.4 (n=8) | 23.0 ±9.9 (n=7) |
| (c) Ticlopidine-HCl | - | 49.3 ±4.3* (n=6) | 33.9 ±7.0 (n=7) | 37.2 ±10.5 (n=9) | - |
| (d) Diltiazem-HCl | - | 56.3 ±8.5* (n=6) | 38.3 ±7.3 (n=7) | - | - |
| No administration (Control group) | 31.4 ± 4.4 (n=16) | | | | |

note 1) n: number of animals used; ** : P < 0.01; * : P < 0.05; (*) : P < 0.1 (significant difference as compared with control)

As is seen in from the above Table, administration of compound (I) brings about a more immediate and lasting anti-platelet action as compared with the other drugs. Therefore, the drug of the present invention can be effectively used as an anti-platelet drug and anti-thrombotic drug, especially where adhesion and aggregation of platelets are concerned.

(ii) Protective action on death caused by endotoxin shock

(Introduction)

Control of platelet functions such as inhibition of platelet aggregation can improve insufficiency of circulation caused by endotoxin to reduce mortality.

(Experimental method)

Compound (I) and ifenprodil tartrate were orally administered to mice and the protective action on death due to endotoxin shock was examined in accordance with a known method (Folia pharmacol. japon, vol. 86, page 17, 1985). The control group was drug-untreated.

(Results and discussion)

Results of the above experiments are shown in Table 5-2.

Table 5-2 note 1)

| Group | Dose (mg/kg) | Number of animals | Number of survival after administration of endotoxin | | | | | |
|---|---|---|---|---|---|---|---|---|
| | | | 18 | 20 | 22 | 24 | 48 hr. | |
| Control group | | 15 | 14 | 9 | 6 | 3 | 0 | |
| Compound (I) | 70 | 15 | 12 | 9 | 8 | 5 | 1 | ns |
| | 200 | 15 | 13 | 13 | 10 | 7 | 5 | * |
| Ifenprodil tartrate | 70 | 14 | 11 | 9 | 6 | 2 | 0 | ns |
| | 200 | 14 | 11 | 9 | 7 | 7 | 5 | ns |

note 1) ns = not significant (no significant difference as compared with control)
$*$ : $P < 0.05$ (significant difference over control)

The above results show that a remarkable protective action on the death is recognized when 200 mg/kg of compound (I) is administered while ifenprodil tartrate exhibits no such effect.

(iii) Inhibitory action on platelet aggregation

(Introduction)

In our experiments, it is confirmed that the anti-thrombotic effect of compound (I) is based on its anti-platelet aggregative activity. In this experiment, further confirmation of this activity and examination of the mechanism involved are studied.

(Experimental method)

The activity of compound (I) (lactate) and ifenprodil tartrate against the fluidity of platelet membrane was examined according to a known method (Biochimica et Biophysica Acta, vol. 798, page 210, 1984) using platelets of rabbit and using n-butyl alcohol as a positive control.

(Results and discussion)

Results of the above experiments are shown in Table 5-3.

It is understood from these results that application of compound (I) (lactate) and n-butyl alcohol causes an increase in the fluitidy of platelet membrane, while ifenprodil tartrate has no such

activity. Therefore, it is confirmed that the mechanism of the action of compound (I) is different from that of ifenprodil and that compound (I) exhibits an inhibitory action on platelet aggregation by increasing the fluidity of platelet membrane.

Table 5-3 note 1)

| Test compound | Fluorescence polarization | | | | | | |
|---|---|---|---|---|---|---|---|
| | Concentration of drug (μM) | | | | | | |
| | 1 | 10 | 21 | 30 | 42 | 62 | 100 |
| Compound (I) | 2±4 | 0±8 | | -2±0 | | | -22±7* |
| n-Butyl alcohol | | | 7±5 | | 6±5 | -14±2* | |
| Ifenprodil tartrate | 7 | 13±7 | | | | | 3±3 |

note 1) Fluidity of platelet membrane increases with the decrease in the value of fluorescence polarization.
* : $P < 0.05$ (significant difference as compared with control)

Test Example 6

(Experimental method)

Experiments were carried out in accordance with Test Example 2 using the saphenous vein in place

of the basilar artery and femoral artery.

In this experiment, phenylephrine and clonidine were used as $\alpha_1$-agonist and $\alpha_2$-agonist, respectively, and these agonists were cumulatively applied to obtain a dose-response curve. After confirming the stabilization of this dose-response curve, each sample was treated for 15 minutes with the following three test compounds and similarly, their dose-response curves were obtained to determine the $pA_2$ value and $pD_2'$ value.

A : Compound (I) (lactate)
B : Ifenprodil (tartrate)
C : Prazosin (hydrochloride)

(Results and discussion)

The results are shown in Table 6.

Table 6[note 1) ] $pA_2$ value[note 2)] and $pD_2'$ value[note 3)]

| Test compound | $\alpha_1$ - agonist: phenylephrine | $\alpha_2$ - agonist: clonidine |
|---|---|---|
| A Compound (I) (lactate) | $pA_2$ = 7.03 ± 0.04 | Note 4) $pD_2'$ = 4.87 |
| B Ifenprodil (tartrate) | 8.22 ± 0.29 | 8.31 |
| C Prazosin (hydrochloride) | 7.93 ± 0.11 | 6.83 |

note 1) The values are expressed by mean value

(n=2) and mean value±standard error.

note 2) The logarithm of the reciprocal of the concentration of inhibiting agent which necessitates doubling the concentration of the agonist in order to keep the effect constant.

note 3) Negative logarithm of dose of inhibiting agent which inhibits maximum response of agonist by 50 %.

note 4) $pA_2$ value an not be obtained.

As may be seen from the above results, ifenprodil and prazosin exhibit a competive antagonism on phenylephrine ($\alpha_1$-agonist) and clonidine ($\alpha_2$-agonist). Ifenprodil exhibits nearly equal competitive antagonism on $\alpha_1$- and $\alpha_2$-agonists while prazosin showes a selective antagonism on the $\alpha_1$-agonist.

On the other hand, compound (I) exhibits a competitive antagonism on the $\alpha_1$-agonist about 1/15 that of ifenprodil and about 1/8 that of prazosin (compared by $pA_2$ value) and exhibits only non-competitive antagonism on the $\alpha_2$-agonist at the very high concentration of $10^{-5}$ M. (No competitive antagonism is recognized even at the concentration of $10^{-6}$ M). Compound (I) exhibits an α-adrenoceptor blocking action on the saphenous vein, activity being specifically higher on the $\alpha_1$-adrenoceptor than prazosin.

From the above results, it is seen that the drug of the present invention can be effectively used as an $\alpha_1$-blocker and anti-hypertensive drug acting specifically on the $\alpha_1$-adrenoceptor.

Test Example 7

(i) Ameliorative activity against damage of cerebral hypoxia

(Experimental method)

Thirty mg/kg each of compound (I) (lactate) and ifenprodil tartrate and 16 mg/kg of phentolamine, which is an α-adrenoceptor blocker, were intraperitoneally administered to ddY male mice, respectively. After 30 minutes from administration, the animals were put in a desiccator and the internal pressure was reduced to 160 mmHg over a period of 6 seconds. Time from start of reduction of pressure until stop of respiration in the mice was measured. The control group was drug-untreated.

(Results and discussion)

The results are shown in Table 7-1.

Table 7-1 note 1)

| Test compound | Number of animals | Time required for stoppage of respiration (sec) |
|---|---|---|
| Control group | 15 | 87.5 ± 9.7 |
| Compound (I) (lactate) | 15 | 126.1 ± 12.1* |
| Ifenprodil tartrate | 14 | 85.2 ± 4.1 |
| Phentolamine | 10 | 54.5 ± 2.9* |

note 1) * : $P < 0.05$ (significant difference as compared with control)

The above results show that compound (I) markedly prolongs survival time, while ifenprodil tartrate has substantially no effect and phentolamine shortens it.

(ii) Prolongation of gasping time in complete cerebral ischemic animals.

(Experimental method)

Compound (I) (lactate), ifenprodil tartrate, calcium hopantenate and glucose were intraperitoneally administered to ddY male mice (10 animals for one group), respectively. After 30 minutes from administration, the mice were decapitated and the duration time of gasping was measured. The control group was drug-untreated.

(Results and discussion)

Results of the experiments are shown in Table 7-2.

The results show that compound (I) dose-dependently prolongs the duration time of gasping, and that the 30 mg/kg compound (I) - treated group shows a prolongation of 116.3 % as compared with the control group. This is an extremely potent action as compared with the 28.7 % prolongation in case of 30 mg/kg of ifenprodil tartrate which is the most potent among the other reference drugs.

Therefore, it is confirmed that the drug of the present invention can be effective in cerebrovascular and cerebral metabolism diseases and so can be used as a cerebrovascular drug and for the improvement and activation of cerebral metabolism.

Table 7-2 note 1)

| Test compound/Dose | Duration time of gasping (sec) | Ratio of time (%) |
|---|---|---|
| Control group | 20.9 ± 1.0 | - |
| Compound (I) (lactate) | | |
| 3 mg/kg | 21.7 ± 0.4 | 3.8 |
| 10 | 26.7 ± 1.8** | 27.8 |
| 30 | 45.2 ± 3.5*** | 116.3 |
| Ifenprodil tartrate | | |
| 10 mg/kg | 23.0 ± 0.5(*) | 10.0 |
| 30 | 26.9 ± 1.2** | 28.7 |
| Calcium hopantenate | | |
| 300 mg/kg | 23.1 ± 0.6(*) | 10.5 |
| Glucose | | |
| 5.4 g/kg | 23.1 ± 0.6(*) | 10.5 |

note 1) *** : $P < 0.001$; ** : $P < 0.01$;
* : $P < 0.05$; (*) : $P < 0.1$
(significant difference as compared with control)

Test Example 8

(Experimental method)

Using the isolated basilar artery and femoral artery treated in accordance with Test Example 2, the activity of (a) compound (I) (lactate), (b) ifenprodil tartrate and (c) verapamil hydrochloride against $^{45}Ca$ influx and $^{45}Ca$ uptake were examined by the conventional method (J. Membran. Biol., vol. 59, page 19, 1981).

(Results and discussion)

Results of the above experiments are shown in Tables 8-1, 8-2 and 8-3.

The results show that compound (I) has the action of inhibiting the increase of $^{45}Ca$ influx and $^{45}Ca$ uptake caused by K and $PGF_{2\alpha}$. That is, it is confirmed that compound (I) inhibits the flowing of Ca into cells through potential-sensitive channels in the vascular smooth muscle.

Table 8-1 note 1)

Inhibitory action on K-induced $^{45}Ca$ influx in isolated femoral artery (n mol/g wt. tissue)

| Test compound/Dose | Pre-incubation time with test compound (min.) | | |
|---|---|---|---|
| | 15 | 45 | 135 |
| Control group | 65.4 ± 4.3** | 41.6 ± 1.4*** | |
| 50 mM KCl | 94.6 ± 5.2 | 98.4 ± 6.8 | |
| + $10^{-5}$ M Ifenprodil tartrate | 99.8 ± 6.4 | 59.0 ± 9.8** | 64.8 ± 7.5** |
| + $10^{-5}$ M Compound (I) lactate | 106.2 ± 4.1 | 38.2 ± 2.6*** | 28.3 ± 1.1*** |
| + $10^{-5}$ M Verapamil hydrochloride | 76.7 ± 2.0* | 29.8 ± 2.8*** | 64.1 ± 5.0** |

note 1) * : $P < 0.05$; ** : $P < 0.01$; *** : $P < 0.001$
(significant difference as compared with 50 mM KCl group. $n = 5 - 7$ .)

Table 8-2 note 1) Inhibitory action on K-induced $^{45}Ca$ uptake in isolated femoral artery (n mol/g wt. tissue)

| Test compound/Dose | |
|---|---|
| Control group | 222.2 ± 11.8*** |
| 50 mM KCl | 586.0 ± 17.9 |
| + $10^{-5}$ M Ifenprodil tartrate | 635.2 ± 41.4 |
| + 5 x $10^{-5}$ M " | 471.2 ± 40.0* |
| + $10^{-4}$ M " | 486.5 ± 20.9* |
| + $10^{-6}$ M Compound (I) lactate | 724.4 ± 32.4* |
| + $10^{-5}$ M " | 461.4 ± 26.7* |
| + $10^{-4}$ M " | 378.8 ± 27.9*** |
| + $10^{-5}$ M Verapamil hydrochloride | 344.7 ± 14.4*** |

note 1) * : $P < 0.05$; ** : $P < 0.01$; *** $P < 0.001$
(significant difference as compared with 50 mM KCl group.
n = 4 - 5 .)

Table 8-3 note 1) Inhibitory action on $PGF_{2\alpha}$-induced $^{45}Ca$ uptake in isolated basilar artery (n mol/g wt. tissue)

| Test compound/Dose | |
|---|---|
| Control group | 134.6 ± 8.3* |
| $10^{-5}$ M $PGF_{2\alpha}$ | 166.2 ± 6.0 |
| + $10^{-5}$ M Compound (I) lactate | 135.2 ± 12.7 (*) |
| + $10^{-5}$ M Ifenprodil tartrate | 125.7 ± 5.6** |
| + $10^{-5}$ M Verapamil hydrochloride | 134.0 ± 4.1** |

note 1) (*) : $P < 0.1$; * : $P < 0.05$; ** : $P < 0.01$ (significant difference as compared with $10^{-5}$ M $PGF_{2\alpha}$ group. n = 4 .)

Test Example 9

(Experimental method)

25 mg/kg of compound (I) was orally administered to three male mice. After 30 minutes from administration, 0.01 mg/kg of isoproterenol, which is a β-agonist, was intravenously administered, and the effect of compound (I) on tachycardia induced by isoproterenol was observed. The control group was compound (I) - untreated.

(Results and discussion)

The results are shown in Table 9.

Table 9

| Test compound | Inhibition % of isoproterenol-induced tachycardia |
|---|---|
| Control group | 0 |
| Compound (I) | 23 |

The above results show that the drug of the present invention has an inhibitory action on stimulation of the β- adrenoceptor and so can be used as an anti-arrhythmic drug and a drug for the treatment of angina pectoris.

Test Example 10

(Experimental method)

Three male mice were fed high cholesterol food for 7 days and 200 mg/kg of compound (I) was orally administered on the 6th day and the 7th day. After a fast over night from the 7th day, measurements

of the total cholesterol in serum (Abell et al, "Standard Method of Clinical Chemistry", vol. 2, page 26, 1985, published by Academic Press; Kanai et al, "Rinsho Shikenho Teiyo", vol. 7, page 64, 1978, published by Kanehara Shuppan) and the amount of β-lipoprotein (Fried-Hoeflmayer, Klin. Wschr., vol. 41, page 246, 1963; Kanai et al, "Rinsho Kensaho Teiyo", vol. 7, page 79, 1978, published by Kanehara Shuppan) were conducted. The control group was compound (I)-untreated.

(Results and discussion)

Results of the above experiments are shown in Table 10.

The results show that the drug of the present invention has the activity of reducing total cholesterol and β-lipoprotein, and so it can be used for the treatment and prophylaxis of disease of the circulatory system such as athero-sclerosis.

Table 10

| Test compound | Reduction % as compared with control group | |
|---|---|---|
| | Total cholesterol | β-lipoprotein |
| Control group | 0 | 0 |
| Compound (I) | 8 | 18 |

Formulation examples of the pharmaceutical preparation for the drug of the present invention are shown below.

Example 1 (formula for one tablet)

| | |
|---|---|
| Compound (I) | 20 mg |
| Crystalline cellulose | 80 mg |
| Lactose | 48 mg |
| Corn starch | 20 mg |
| Magnesium stearate | 2 mg |
| Total amount | 170 mg |

Example 2 (formula for one ampule of injection)

| | |
|---|---|
| Compound (I) lactate | 5 mg |
| Distilled water for injection | suitable amount to make up 2 ml |
| Total amount | 2 ml |

Example 3 (formula for 100 g of ointment)

| | |
|---|---|
| Compound (I) | 1 g |
| Gelatin | 3 g |
| Hydroxyethyl cellulose | 2 g |
| Sodium polyacrylate | 0.5 g |
| Butylene glycol | 4 g |
| Ethanol | 30 g |
| Purified water to make up 100 g | |
| Total amount | 100 g |

Example of the synthetic method for compound (I) is shown below.

Synthesis of Compound (I)

5.0 g of 1-(4-benzyloxyphenyl)-2-(4-benzylpiperidino)propan-1-one was dissolved in a mixture of 100 ml of methanol and 10 ml of water and thereto was gradually added 2.0 g of sodium borohydride with stirring and under ice-cooling followed by stirring at room temperature for one hour. The methanol was distilled off and the residue was acidified with 10 % hydrochloric acid, then made basic with a saturated aqueous sodium hydrogencarbonate solution and extracted with 20 ml of benzene. The extract was evaporated to give a residue which was subjected to column chromatography using 60 g of silica gel and chloroform as a solvent to isolate the threo form and erythro form.

The threo form was recrystallized from ethanol to obtain 1.1 g (yield 27.4 %) of threo-2-(4-benzylpiperidino)-1-(4-benzyloxyphenyl)propan-1-ol as a colorless prism having a melting point 153 - 154°C.

IR (KBr, $cm^{-1}$) : 3280 (OH)

NMR ($CDCl_3$) δ :

0.70 (3H, d, J=7 Hz, CH-C$\underline{H}_3$)

1.0-3.2 (12H, m)

4.16 (1H, d, J=10 Hz, C$\underline{H}$-OH)

4.5-5.3 (1H, broad s, O$\underline{H}$)

5.00 (2H, s, $C_6H_5C\underline{H}_2O$)

6.6-7.6 (14H, m, aromatic protons)

Elementary analysis

Calcd. for $C_{28}H_{33}NO_2$ : C, 80.92; H, 8.00; N, 3.37

Found : C, 80.77; H, 7.96; N, 3.41

The erythro form was recrystallized from ethanol to obtain 2.1 g (yield 52.2 %) of erythro-2-(4-benzylpiperidino)-1-(4-benzyloxyphenyl)propan-1-ol as a colorless leaflet having a melting point 126 - 127°C.

IR (KBr, $cm^{-1}$) : 3150 (OH)
NMR ($CDCl_3$) δ :
0.81 (3H, d, J=7 Hz, CH-C$\underline{H}_3$)
1.1-3.2 (12H, m)
3.67 (1H, broad s, O$\underline{H}$)
4.74 (1H, d, J=5 Hz, C$\underline{H}$-OH)
4.99 (2H, s, $C_6H_5C\underline{H}_2O$)
6.7-7.6 (14H, m, aromatic protons)

Elementary analysis
Calcd. for $C_{28}H_{33}NO_2$ : C, 80.92; H, 8.00; N, 3.37
Found : C, 80.96; H, 8.10; N, 3.27

Industrial Applicability

As is clear from the above disclosures, the present invention has provided a cardiovascular drug which has various circulation improving actions (cerebral circulation improving action, vasodilative action, coronary vasodilative action, calcium antagonistic action, $\alpha_1$-blocking action, anti-thrombotic action, etc.) and which is especially superior in its effective time, effectiveness and specificity of the activity. Such drug of the present invention can be used for treatment, prophylaxis and improvement of above mentioned various lesions, disorders and diseases of the circulation systems and is a drug valuable in medical care.

## CLAIMS

1. A cardiovascular drug comprising 2-(4-benzylpiperidino)-1-(4-benzyloxyphenyl)propan-1-ol or its salt as the active ingredient.

2. A cardiovascular drug according to claim 1 which is a cerebrovascular drug.

3. A cardiovascular drug according to claim 1 which is a vasodilator.

4. A cardiovascular drug according to claim 1 which is a coronary vasodilator.

5. A cardiovascular drug according to claim 1 which is a calcium antagonist.

6. A cardiovascular drug according to claim 1 which is an anti-platelet aggregation drug.

7. A cardiovascular drug according to claim 1 which is an $\alpha_1$-blocker.

8. A cardiovascular drug according to claim 1 wherein the salt of 2-(4-benzylpiperidino)-1-(4-benzyloxyphenyl)propan-1-ol as an active ingredient is a pharmaceutically acceptable salt of an aliphatic carboxylic acid having 2-20 carbon atoms, an aliphatic or aromatic sulfonic acid having 1-10 carbon atoms or an inorganic mineral acid.

9. A cardiovascular drug according to claim 1 wherein the active ingredient is erythro-2-(4-benzylpiperidino)-1-(4-benzyloxyphenyl)propan-1-ol or its salt.

10. A cardiovascular drug according to claim 1 which is an oral dosage form, an injection, a mucosal dosage form or a transdermic dosage form.

# INTERNATIONAL SEARCH REPORT

0232423

International Application No. PCT/JP86/00350

**I. CLASSIFICATION OF SUBJECT MATTER** (if several classification symbols apply, indicate all) [3]

According to International Patent Classification (IPC) or to both National Classification and IPC

Int. $Cl^4$ A61K31/445, C07D211/14

**II. FIELDS SEARCHED**

Minimum Documentation Searched [4]

| Classification System | Classification Symbols |
|---|---|
| IPC | A61K31/445, C07D211/14 |

Documentation Searched other than Minimum Documentation to the Extent that such Documents are Included in the Fields Searched [5]

**III. DOCUMENTS CONSIDERED TO BE RELEVANT** [14]

| Category* | Citation of Document, [16] with indication, where appropriate, of the relevant passages [17] | Relevant to Claim No. [18] |
|---|---|---|
| | | |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search [2] | Date of Mailing of this International Search Report [2] |
|---|---|
| October 6, 1986 (06. 10. 86) | October 20, 1986 (20. 10. 86) |
| International Searching Authority [1] | Signature of Authorized Officer [20] |
| Japanese Patent Office | |